**Europäisches Patentamt**

**European Patent Office**

**Office européen des brevets**

(19)

(11) Veröffentlichungsnummer: **0 064 687**
A2

(12) # EUROPÄISCHE PATENTANMELDUNG

(21) Anmeldenummer: 82103693.6

(22) Anmeldetag: 30.04.82

(51) Int. Cl.³: **A 61 B 5/10**
**A 61 C 19/04**

(30) Priorität: 30.04.81 DE 3117174
08.07.81 DE 3126911

(43) Veröffentlichungstag der Anmeldung:
17.11.82 Patentblatt 82/46

(84) Benannte Vertragsstaaten:
AT CH FR IT LI

(71) Anmelder: Klett, Rolf, Dr. Dr.
Nikolaushöhe 15a
D-8702 Gerbrunn(DE)

(72) Erfinder: Klett, Rolf, Dr. Dr.
Nikolaushöhe 15a
D-8702 Gerbrunn(DE)

(74) Vertreter: Reichel, Wolfgang, Dipl.-Ing. et al,
Reichel und Reichel Parkstrasse 13
D-6000 Frankfurt am Main 1(DE)

(54) Vorrichtung zur berührungslosen ein- bis dreidimensionalen Vermessung von Kiefer- bzw. Kiefergelenkbewegungen.

(57) Die Erfindung betrifft eine Vorrichtung zur berührungslosen ein-bis dreidimensionalen Vermessung von Kiefer- bzw. Kiefergelenkbewegungen mit Hilfe von Meßeinrichtungen, die optoelektronische Signalgeber und Signalempfänger aufweisen, zwischen denen Lichtsignale übertragen werden. Bei einer ersten Ausführungsform ist am Unterkiefer ein Meßkopf befestigt, der Lichtsender und Lichtempfänger aufweist und der sich mit dem Unterkiefer gegenüber reflektierenden Meßplatten bewegt. Die Lichtempfänger erzeugen Signale, die sich mit der Länge des Lichtweges zwischen Lichtsender und -empfänger ändern und die somit die Bewegung des Unterkiefers darstellen. Bei einer zweiten Ausführungsform bilden die Lichtsender einen gebündelten Strahl, und sie sind so angeordnet, daß sie sich mit dem Unterkiefer relativ zu Lichtempfängern bewegen, die eine ein- oder zweiachsige Positionsempfindlichkeit quer zur Auftreffrichtung des Lichtstrahls aufweisen. Bei einer dritten Ausführungsform werden die Lichtsender mit dem Unterkiefer relativ zu Gittern bewegt, so daß Lichtimpulse entstehen, die der Bewegung der Unterkiefers entsprechen.

EP 0 064 687 A2

- 1 -

Dr. Dr. Rolf Klett, D-8700 Würzburg

-----------------------------------------------------------------

Vorrichtung zur berührungslosen ein- bis dreidimensionalen Vermessung von Kiefer- bzw. Kiefergelenkbewegungen

-----------------------------------------------------------------

Die Erfindung betrifft Vorrichtungen zur berührungslosen ein- bis dreidimensionalen Vermessung von Kiefer- bzw. Kiefergelenkbewegungen mit Hilfe von Meßeinrichtungen, die optoelektronische Signalgeber und Signalempfänger aufweisen, zwischen denen Lichtsignale direkt oder über zur Meßeinrichtung gehörende Reflektoren übertragen werden, bei der am Unterkiefer des Patienten ein Teil der Meßeinrichtung angebracht ist.

Eine derartige Vorrichtung ist bereits aus der DE-OS 28 25 204 (Seite 12, Zeile 9 bis 14) bekannt. Bei dieser bekannten Vorrichtung sind die in jeweils einer Ebene liegenden Signalempfänger (Sensoren) auf einer Platte angeordnet, und zwar als eine Vielzahl von Fototransistoren, die elektrisch miteinander verbunden sind. Als Lichtquelle werden optoelektronische Signalgeber, wie Fotodioden, verwendet. Eine Vermessung in jeder Raumrichtung ist bei dieser Vorrichtung dadurch möglich, daß das von den Fototransistoren empfangene integrale Signal vom Abstand der Fototransistoren zur Lichtquelle abhängig ist. Der Nachteil dieser Abstandsabhängigkeit besteht darin, daß das Meßsignal insbesondere bei großen Abständen zwischen Signalgeber und Signalempfänger leicht durch Störlicht verfälscht werden kann und daß keine Linearität zwischen Meßsignal und Abstand zwischen Signalgeber und Signalempfänger besteht. Eine Linearisierung ist mit Hilfe von elektronischen Korrekturschaltungen nur mit großem Aufwand und nur näherungsweise erreichbar. Weiterhin ist bei dieser Vorrichtung der Aufbau der Platten mit vielen Signalempfängern bzw. auch mit den Signalgebern verhältnismäßig
kompliziert. Die vielen Empfänger müssen hierbei auf einer möglichst ausgedehnten Fläche angeordnet und parallel
geschaltet sein, damit das integrale Empfangssignal nur
vom Abstand zwischen Sender- und Empfangsfläche und nicht
durch Randzoneneffekte zusätzlich auch noch von der Querverschiebung des Signalgebers (bzw. Reflektors) zur Empfangsebene abhängig ist und somit das abstandsabhängige
Meßergebnis verfälscht werden würde. Da Kiefergelenkbewegungen in verschiedenen Richtungen bis zu ca. 2 cm
möglich sind, müßten daher die Empfangsflächen sehr ausgedehnt sein und viele genau gleichartige Empfänger enthalten, um Homogenität der Empfangsempfindlichkeit über den
gesamten Bewegungsraum und darüber hinaus zu erreichen.
Will man über den gesamten Bewegungsraum unabhängig von
den Querverschiebungen sein, so müssen auch weit außerhalb des Bewegungsraums, dort, wo noch meßbare Lichtintensität vorhanden ist, Empfänger angebracht sein. Ansonsten
verfälschen Randzoneneffekte das Meßergebnis erheblich.

Es ist andererseits aus der DE-OS 28 25 204 (Fig. 4
und dazugehöriger Text) eine Vorrichtung bekannt, bei der
der den Platten mit Signalgebern und Signalempfängern
gegenüberstehende Meßkopf kugelförmig ausgebildet ist und
eine allseitig reflektierende Oberfläche hat, damit Raumdrehungen des Reflektors nicht in das Meßergebnis eingehen. Auch hier sind viele Sender und Empfänger auf einer
großen Fläche verteilt und parallel geschaltet, um das
Ausgangssignal unabhängig von der Querverschiebung des
Reflektors zur Meßfläche zu machen. Diese bekannte Meßvorrichtung ist sehr aufwendig aufgebaut. Will man den
Aufwand und die technischen Probleme durch Verminderung
der Zahl der Signalgeber-Signalempfänger-Einheiten verringern, so ist dies nur durch ein eingeschränktes Meßfeld und ein durch Randzoneneffekte gestörtes Meßsignal
zu erkaufen.

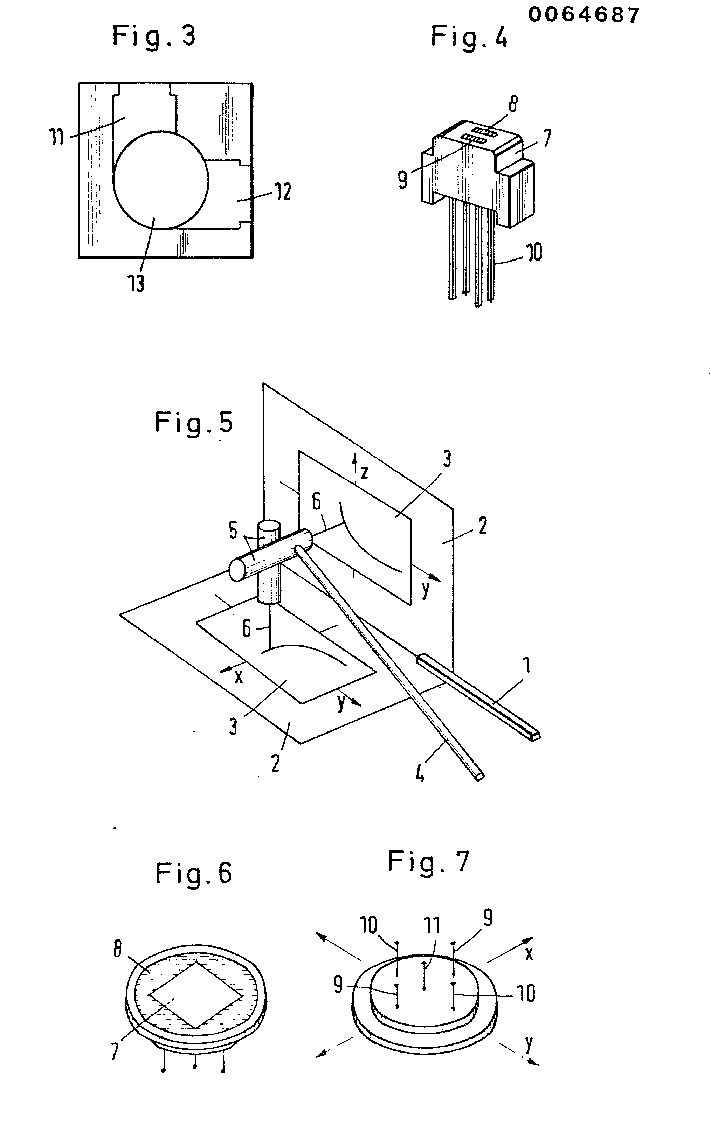

Das Prinzip der abstandsabhängigen Wegmessung, bei
der der Abstand einer Lichtquelle zu der betreffenden
Fotozelle als Maß für die Entfernung dient, wurde im
übrigen bereits früher beschrieben. (J. Pros. Dent.,
Vol. 17, February, 1967, Seiten 109-121). Auch bei
dieser Anordnung werden mehrere Fotozellen nebeneinander angeordnet und parallel geschaltet ( siehe Figur 3
und Figur 4 auf Seite 111 bzw. Seite 113 und zugehöriger Text), damit sich bei einer Bewegung der Lichtquelle auf einer Linie gleichen Abstandes von den Fotozellen keine Änderung des Ausgangssignals ergibt.

Es ist darüber hinaus eine Meßanordnung bekannt
(Dental Clinics of North America, 13, 1969, Seiten
629-642), bei der sechs  "incremental transducers" als
Baueinheiten (Seiten 631 und 632) vorgesehen sind. Bei
jedem "incremental transducer" wird ein Gitter zwischen
Leuchtdioden und Fotozellen bewegt, wobei die Bewegung
des Gitters mechanisch geführt ist. Die Montageenden
jedes "incremental transducers" sind jeweils mit den beiden an Ober- und Unterkiefer befestigten Gesichtsbögen
mechanisch verbunden. Durch diese Verbindung und die
mechanische Führung der Gitter ist die Bewegung von
Ober- und Unterkiefer nicht kräftefrei. Eine für die
Kiefergelenksdiagnostik wesentliche ungestörte Aufzeichnung der Unterkieferbewegung, die durch empfindliche
neuromuskuläre Reflexe gesteuert wird, ist somit unmöglich. Außerdem ist das Empfangssignal jeden Transducers kein direktes Maß für die diagnostisch wichtige
Bewegung der Kiefergelenke in einer vorgegebenen Raumrichtung, weil jeder Transducer bei der Unterkieferbewegung weitab von den Kiefergelenken lediglich den Abstand zwischen den Montagepunkten des Transducers an den beiden Gesichtsbögen mißt. Eine Bewegung am Gelenkort kann entsprechend der Mechanik des starren Körpers nur mit einem aufwendigen Rechenprogramm ermittelt werden.

Der Erfindung liegt die Aufgabe zugrunde, eine Vorrichtung der eingangs erwähnten Art zu schaffen, die einfach aufgebaut ist und keine höheren Anforderungen an die Auswerteschaltung stellt und dabei Ausgangsgrößen abgibt, die ein direktes Maß für die dreidimensionale Verschiebung des Kiefers bzw. Kiefergelenkes darstellen.

Diese Aufgabe wird durch die Merkmale des kennzeichnenden Teils der Ansprüche 1, 8 oder 15 gelöst.

Bei der ersten Form der erfindungsgemäßen Vorrichtung ist es vorteilhaft, daß für jede Ebene nur ein Signalgeber und ein Signalempfänger benötigt wird und somit ein einfacher Aufbau gewährleistet ist. Durch das Prinzip, Sender und Empfänger unmittelbar nebeneinander anzuordnen und den Reflektor flächenförmig auszubilden, wird die Unabhängigkeit des Meßsignals von der Querverschiebung erreicht, weil sich bei der Querverschiebung die Reflexionsbedingungen nicht ändern und weil nur die in sich zurückreflektierten Lichtstrahlen den Empfänger erreichen und gemessen werden. Auch geringe Drehungen des Reflektors bleiben ohne störenden Einfluß, wenn die Strahlungscharakteristik des Senders nicht stark gebündelt ist. Darüber hinaus besteht der besondere Vorteil einer schnellen und sicheren räumlichen Justierung des Meßkopfes, da durch Einschieben des Meßkopfes in die mit den Abstandshalterplatten bestückten Reflektoren, welche in komplementärer Form zum Meßkopf angeordnet sind, eine Selbstzentrierung erfolgt.

Die Fixierung der Raumlage des Meßkopfes oder der Reflektoren geschieht durch Arretierung der Drehgelenke. Dieser Vorgang nimmt nur einige Sekunden in Anspruch. Zur schnellen Nullpunkteinstellung (Grundabstand) sind in ihrer Stärke wählbare Abstandshalter zwischen die reflektierenden Oberflächen der Meßplatte und den Meßkopf einschiebbar. Aufgrund des genau definierbaren Grundabstands lassen sich die systembedingten Linearitätsgrenzen der Vorrichtung sehr gut einhalten.

Die erfindungsgemäße Vorrichtung zur berührungslosen ein- bis dreidimensionalen Vermessung von Kiefer- und Kiefergelenkbewegungen nach Anspruch 8 weist als wesentliches Merkmal einen Signalempfänger mit einer Positionsempfindlichkeit quer zur Auftreffrichtung des Lichtstrahls auf.

Durch die erfindungsgemäße Anordnung nach Anspruch 8 ergeben sich mehrere Vorteile. Bei Verwendung handelsüblicher ein- oder zweiachsiger positionsempfindlicher Signalempfänger, deren wesentliches Merkmal die meist von Haus aus vorhandene Linearität zwischen der Positionsverschiebung des auftreffenden Lichtstrahls und dem Ausgangssignal ist, erreicht man ohne komplizierten und besonderen schaltungstechnischen Aufwand eine sehr gute Linearität zwischen Meßsignal und Relativbewegung des Signalempfängers quer zum Lichtstrahl, so daß eine elektronische Linearisierungsschaltung nicht mehr nötig ist. Der Linearitätsbereich reicht dabei bis zu einigen Zentimetern. Größere Linearitätsbereiche sind für die Diagnostik von Kiefer- bzw. Kiefergelenkbewegungen nicht erforderlich. Dabei wird ein hohes Auflösungsvermögen besser als 10 μm erreicht. Insbesondere liegt eine nachteilige Beeinflussung des Ausgangssignals von Abstandsänderungen zwischen Lichtquelle und Signalempfänger nicht vor. Ein besonderer Vorteil bei der Verwendung eines zweiachsigen positionsempfindlichen Signalempfängers liegt darin, daß mit einem Sender und einem Empfänger eine präzise und unkomplizierte Vermessung bereits in zwei Raumdimensionen möglich ist und daß die Ausgangssignale des Signalempfängers ein direktes Maß für die Verschiebung in den betreffenden Raumrichtungen sind. Bei den handelsüblichen positionsempfindlichen Signalempfängern läßt sich durch bekannte Schaltungsmaßnahmen relativ einfach eine wirkungsvolle Fremdlichtkompensation durchführen.

Eine sehr gute Linearität und ein hohes Auflösungsvermögen lassen sich bei der Vorrichtung nach

- 7 -

Anspruch 8 auch erreichen, wenn als lichtempfindliche Signalempfänger beispielsweise Quadrantenfotodetektoren oder kontinuierlich messende lineare positionsempfindliche Dioden mit ein- oder zweiachsiger Positionsempfindlichkeit verwendet werden, deren analoge Ausgangssignale den Positionsverschiebungen des auftreffenden Lichtstrahls proportional sind.

Für digitale Meßwerterfassung sind Ladungsverschiebungsvorrichtungen, z. B. ladungsgekoppelte Vorrichtungen (CCD), besonders vorteilhaft. Diese Vorrichtungen sind in winzige diskrete lichtempfindliche Flächenelemente (ca. 10 µm x 10 µm) unterteilt. Die Ausgangssignale werden wie bei Bildsensoren verarbeitet und stehen in digitaler Form zur Verfügung, was die Verarbeitung der Signale mit Hilfe von Rechnern sehr vereinfacht.

Bei der Lösung nach Anspruch 15 werden bei der Bewegung des Unterkiefers die Striche eines Gitters quer zur Strichrichtung optisch abgetastet und abgezählt. Wesentlich ist, daß dabei keine direkte oder indirekte Kraft zwischen Signalgebern und Gittern übertragen wird. Die Zahl der empfangenen Impulse ist ein direktes Maß für die Größe der Kieferbewegung in einer Richtung quer zum Gitter. Der Vorteil dieses Lösungsweges ist die durch die Qualität des Gitters bestimmte Linearität und die Tatsache, daß die Signale digital vorliegen und sofort in Rechnern oder Zählern weiter verarbeitet werden können. Bei der Verwendung von kodierten Gittern lassen sich vorteilhaft auch absolute Positions-messungen durchführen.

Für die Erzeugung eines gebündelten Lichtstrahls eignen sich vorzugsweise Leuchtdioden mit enger Ausstrahlcharakteristik. Deren ausgesandtes Licht kann durch Linsensysteme noch besser gebündelt werden. Beste Ergebnisse hinsichtlich des Auflösungsvermögens lassen

- 8 -

sich durch Verwendung von Lasern erreichen (z.B.vom Helium-Neon-Typ), da diese einen parallelen Lichtstrahl abgeben. Einer Gefahr der Augenverletzung kann durch Abschirmung begegnet werden.

Die verschiedenen Vorrichtungen zur berührungslosen ein- bis dreidimensionalen Vermessung von Kiefer- bzw. Kiefergelenkbewegungen sind ohne weiteres mit gebräuchlichen Pantographen kombinierbar, die mit Schreibtafeln und Schreibsticheln arbeiten, wie sie in gnathologisch ausgerichteten Zahnarztpraxen vorhanden sind. Diese komplizierten mechanischen Geräte sind sehr teuer und können durch die erfindungsgemäße Meßvorrichtung preiswert zu hochpräzisen Instrumenten ergänzt werden. Der besondere Vorteil liegt weiterhin darin, daß die in teuren Fortbildungskursen erlernte Handhabung der Geräte nicht wesentlich geändert werden muß. Vielmehr vereinfacht sich bei wesentlich steigender Meßinformation der Justiervorgang.

Ausführungsformen der Erfindung werden nachstehend anhand der Zeichnungen beispielshalber beschrieben. Dabei zeigen:

Fig. 1 eine perspektivische Ansicht der Meßvorrichtung nach der Erfindung nach Anspruch 1,

Fig. 2 eine perspektivische Ansicht des Meßkopfes der Meßvorrichtung nach Fig. 1, wobei der Meßkopf gegenüber seiner Arbeitslage um 180$^\circ$ gedreht ist,

Fig. 3 einen Schnitt durch den Meßkopf nach Fig. 2 längs der Ebene A-A,

Fig. 4 eine integrierte Signalgeber-/Signalempfängeranordnung, wie sie in einem Meßkopf nach Fig. 2 und 3 verwendbar ist,

- 9 -

Fig. 5 eine Vorrichtung zur berührungslosen dreidimensionalen Vermessung von Kiefer- bzw. Kiefergelenkbewegungen mit Hilfe von zweiachsigen positionsempfindlichen Fotodetektoren nach Anspruch 8,

Fig. 6 eine Ausführungsform einer zweiachsigen linearen positionsempfindlichen Diode als lichtempfindlicher Signalempfänger schräg von oben,

Fig. 7 eine Diode nach Fig. 6 schräg von unten,

Fig. 8 die Übertragungsfunktion der positionsempfindlichen Diode nach Fig. 6 in einer Achsenrichtung,

Fig. 9 eine Prinzipschaltung zur Auswertung der von einem lichtempfindlichen Signalempfänger mit zweiachsiger Positionsempfindlichkeit abgegebenen Signale und

Fig. 10 eine Vorrichtung zur berührungslosen dreidimensionalen inkrementalen Vermessung von Kiefer- bzw. Kiefergelenkbewegungen nach Anspruch 15.

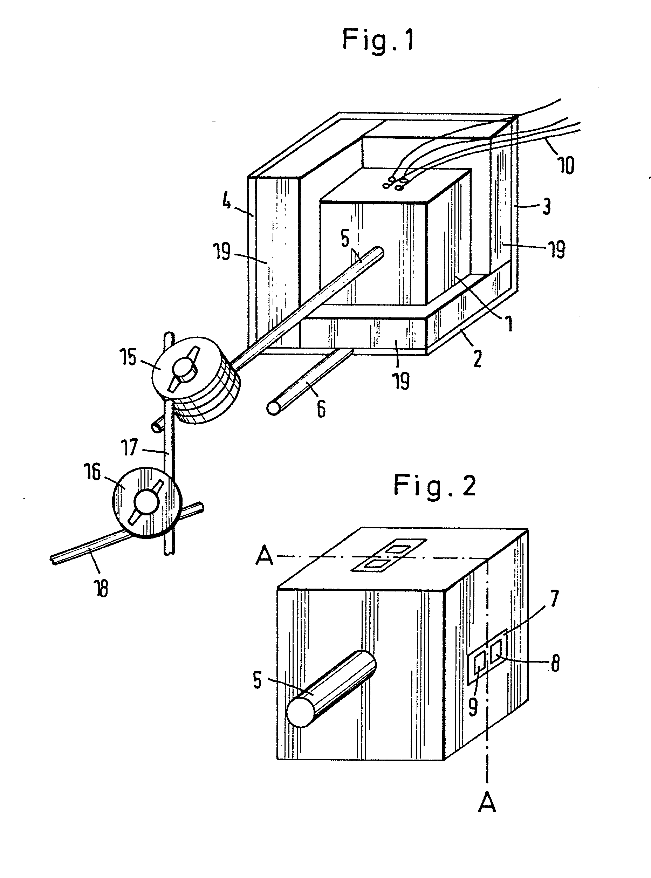

Die in Fig. 1 dargestellte Meßvorrichtung weist einen Meßkopf 1 auf, dem senkrecht zueinander angeordnete Meßplatten 2, 3, 4 gegenüberstehen. Der Meßkopf 1 ist an einem Arm 5 gehaltert, der über zwei Drehgelenke 15 und 16 und eine Zwischenstange 17 mit der Querstange 18 eines Pantographen verbunden ist, die am Unterkiefer eines Patienten befestigt ist. Die Meßplatten 2, 3 und 4 sind miteinander verbunden und werden durch einen Arm 6 gehaltert, der über ein Gestänge des Pantographen mit dem Oberkiefer des Patienten verbunden ist. Die dem Meßkopf gegenüberliegenden Oberflächen der Halterungsplatten 2, 3 und 4 weisen optisch reflektierende Oberflächen auf. In die den reflektierenden Oberflächen der Meßplatten 2, 3 und 4 gegenüberliegenden Oberflächen des Meßkopfes sind integrierte optische Sender- und Empfängereinheiten 7 eingelassen, die so ausgerichtet sind, daß das von dem Sender 8 abgegebene Licht durch die gegenüberliegende Reflexionsfläche zu dem Empfänger 9 reflektiert wird. Als Sender 8 wird beispielsweise eine lichtemittierende Diode verwendet. Als Empfänger 9 wird beispielsweise ein Fototransistor verwendet, dessen Ausgangsstrom von der Größe des Lichtweges zwischen Sender und Empfänger abhängig ist. Am oberen Ende des Meßkopfes 1 sind die Zuleitungsdrähte 10 für Sender 8 und Empfänger 9 herausgeführt. Diese werden mit einer Auswerteschaltung verbunden.

Zur Justierung wird der würfelförmige Meßkopf 1 in die Anordnung aus den drei senkrecht aufeinander angeordneten Meßplatten 2, 3 und 4 mit den senkrecht aufeinander stehenden Reflexionsflächen eingeschoben. Die dabei sich einstellende Lage des Meßkopfes 1 gegenüber den Reflexionsflächen der Meßplatten 2, 3 und 4 wird fixiert. Dies erfolgt durch einfache Arretierung mit Hilfe eines Doppelgelenks, das zwischen Unterkieferquerstange 18 des Pantographen und Arm 5 des Meßkopfes vorgesehen ist.

Um den Beginn des Meßverlaufes festzulegen, werden bei der Justierung zwischen die Reflexionsflächen und den Meßkopf in ihrer Dicke frei wählbare Abstandsplatten 19 eingeschoben, die nach Arretierung des Doppelgelenks herausgezogen werden. Es können dann die Messungen sofort ausgeführt werden.

Der Meßkopf kann gegenüber den Reflexionsflächen um mehrere Winkelgrade gekippt werden, ohne daß sich das Ausgangssignal wesentlich ändert.

Vor der Messung werden die Meßplatten auf die Drehachse des Kiefergelenks ausgerichtet.

Mit Hilfe der Meßvorrichtung ist die Relativbewegung zwischen Unterkiefer und Oberkiefer meßbar.

Die erfindungsgemäße Meßvorrichtung ist in einfacher Weise direkt in üblicherweise verwendeten Pantographen, die mit Schreibplatten und Schreibstiften arbeiten, verwendbar, wobei die erfindungsgemäße Meßvorrichtung anstelle der Schreibplatte und Schreibstifte direkt an das vorhandene Gestänge der bekannten Pantographen angesetzt werden kann. Das mit der erfindungsgemäßen Meßvorrichtung erreichte Auflösungsvermögen ist mit circa 10 $\mu$m wesentlich höher als die mit den Schreibplatten oder Schreibstiften erreichte Genauigkeit.

Die in Fig. 2 dargestellte Ansicht des Meßkopfes nach Fig. 1 zeigt die Anordnung der integrierten Sender- und Empfängereinheiten 8,9.

- 12 -

Der in Fig. 3 dargestellte Schnitt längs der Schnittebene A-A zeigt, daß der Meßkopf 1 Ausnehmungen 11 und 12 aufweist, in die Sender- und Empfängereinheiten 7 einsetzbar sind. Ferner befindet sich in der Mitte des Meßkopfes 1 eine Ausnehmung 13, durch die die Anschlußleitungen 10 nach außen geführt werden.

Eine Sender- und Empfängereinheit 7, wie sie bei dem Meßkopf nach den Fig. 2 und 3 verwendbar ist, ist in Fig. 4 dargestellt. Man erkennt, daß der Sender 8 unmittelbar neben dem Empfänger 9 angeordnet ist. Die Anschlußleitungen 10 sind auf der Gegenseite der Einheit herausgeführt.

Die in Fig. 5 dargestellte Vorrichtung zur berührungslosen dreidimensionalen Vermessung von Kiefer- bzw. Kiefergelenkbewegungen ist in der folgenden Weise aufgebaut. Mit dem Oberkiefer ist eine Stange 21 verbunden, an der zwei senkrecht aufeinanderstehende Halterungsteile 22 befestigt sind, auf denen jeweils ein lichtempfindlicher Signalempfänger 23 mit zweiachsiger Positionsempfindlichkeit angebracht ist. Die Signalempfänger mit zweiachsiger Positionsempfindlichkeit geben jeweils in zwei Richtungen, nämlich x und y bzw. z und y ein Signal zur Auswertung in eine Auswerteschaltung ab. Am Unterkiefer ist eine Stange 24 befestigt, an der Signalgeber 25 angebracht sind. Die Signalgeber 25 geben jeweils einen gebündelten Lichtstrahl 26 ab, der senkrecht auf die lichtempfindlichen Signalempfänger 23 auftrifft. Bei Verschiebung oder Drehung des Unterkiefers gegenüber dem Oberkiefer wird die quer zur Auftreffrichtung des Lichtstrahls veränderte Position durch die Signalempfänger fest gehalten und von dem betreffenden Signalempfänger ein x-, y-Signal oder ein y-. z-Signal abgegeben.

In Fig. 6 ist eine zweiachsige lineare positionsempfindliche Diode schräg von oben dargestellt. Man er-

- 13 -

kennt das quadratische positionsempfindliche Gebiet 27
inmitten der Abdeckung 28.

In Fig. 7 ist die zweiachsige lineare positionsempfindliche Diode von der Anschlußseite dargestellt.
Sie weist für die beiden senkrecht aufeinanderstehenden
Achsenrichtungen x und y jeweils zwei Anschlüsse 29
bzw. 30 auf. Der Mittelanschluß 31 dient zur Spannungsversorgung.

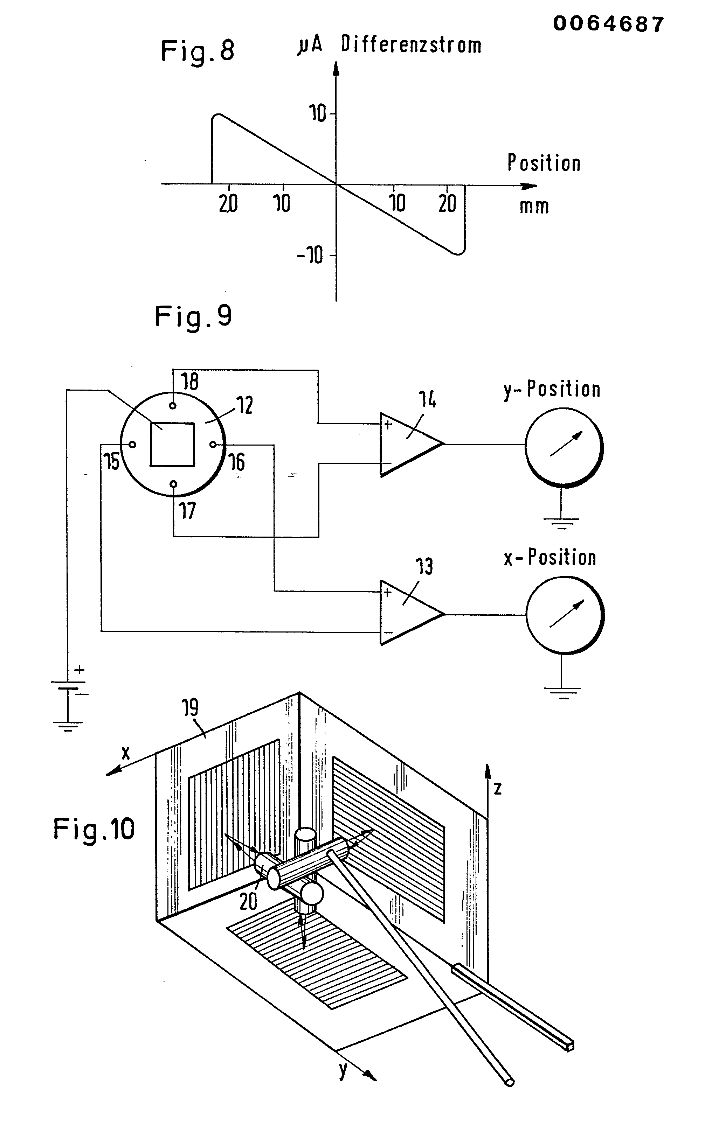

In Fig. 8 ist die Übertragungsfunktion einer linearen positionsempfindlichen Diode für jede Achse dargestellt. Wie man erkennt, ist die Übertragungsfunktion
für einen weiten Bereich der Positionsverschiebung des
auftreffenden Lichtstrahls linear. Auf diese Weise läßt
sich mit zwei zweiachsigen linearen positionsempfindlichen Dioden eine exakte Messung mit einer Vorrichtung
nach der in Fig. 5 dargestellten Art vornehmen.

In Fig. 9 ist eine Prinzipschaltung zur Auswertung
der Ausgangssignale einer zweiachsigen linearen positionsempfindlichen Diode dargestellt. Diese Schaltung
weist eine lineare zweiachsige positionsempfindliche
Diode 32 auf, deren Anschlüsse 35 und 36 für die x-
Richtung mit einer Differenzbildungsschaltung 33 und
deren Anschlüsse 37 und 38 für die y-Richtung mit einer
weiteren Differenzbildungsschaltung 34 verbunden sind.
Aus der Differenz der Ströme an den Anschlüssen 35 und 36
ergibt sich unmittelbar die x-Position und aus der Differenz der Ströme an den Anschlüssen 37 und 38 ergibt sich
unmittelbar die y-Position. Die Ausgangssignale können
dann direkt zur Anzeige gebracht oder mit Hilfe eines
Rechners weiter verarbeitet werden.

Die in Fig. 10 dargestellte Vorrichtung weist in
drei zueinander senkrechten Raumebenen Strichgitter 39

- 14 -

auf. Von Sendern des Meßkopfes 40 werden senkrecht auf
die Ebene Lichtstrahlen ausgesandt, die von den Gittern
auf den Empfänger im Meßkopf 40 reflektiert werden, so
daß digitale Empfängersignale entstehen. Bei dieser Vorrichtung sind die Gitter mit dem Oberkiefer und der Meßkopf mit dem Unterkiefer verbunden.

- 15 -

Patentansprüche

1. Vorrichtung zur berührungslosen ein- bis dreidimensionalen Vermessung von Kiefer- bzw. Kiefergelenkbewegungen mit Hilfe von Messeinrichtungen, die optoelektronische Signalgeber und Signalempfänger aufweisen, zwischen denen Lichtsignale direkt oder über zur Messeinrichtung gehörende Reflektoren übertragen werden, bei der am Unterkiefer des Patienten ein Teil der Messeinrichtung angebracht ist,
d a d u r c h   g e k e n n z e i c h n e t,
dass in den drei Ebenen verlaufende Messplatten (2, 3, 4) jeweils eine reflektierende Oberfläche gegenüber einem Messkopf aufweisen und dass der den reflektierenden Oberflächen gegenüberstehende Messkopf (1) gegenüber jeder der Flächen sowohl einen Sender (8) als auch einen Empfänger (9) aufweist, der das von der gegenüberliegenden Fläche reflektierte Signal aufnimmt.

2. Vorrichtung nach Anspruch 1,
d a d u r c h   g e k e n n z e i c h n e t,
dass die Messplatten (2, 3, 4) in oder parallel zu drei senkrecht zueinander stehenden Schädelebenen (horizontal, frontal und sagittal) vorgesehen sind.

3. Vorrichtung nach Anspruch 1,
d a d u r c h   g e k e n n z e i c h n e t,
dass jeder Sender (8) und jeder Empfänger (9) nebeneinander in den Messkopf (1) eingesetzt sind.

4. Vorrichtung nach Anspruch 3,
d a d u r c h   g e k e n n z e i c h n e t,
dass der Sender (8) und Empfänger (9) als integrierter Baustein ausgebildet sind.

- 16 -

5. Vorrichtung nach einem der vorhergehenden Ansprüche,
d a d u r c h   g e k e n n z e i c h n e t,
dass der Messkopf (1) würfelförmig ausgebildet ist.

6. Vorrichtung nach einem der vorhergehenden Ansprüche,
d a d u r c h   g e k e n n z e i c h n e t,
dass bei der Justierung Flächenkontakt zwischen den reflektierenden Oberflächen der Messplatten (2, 3, 4) der
jeweils zugehörigen Oberfläche des Messkopfes (1) vorliegt.

7. Vorrichtung nach Anspruch 6,
d a d u r c h   g e k e n n z e i c h n e t,
dass bei der Justierung Abstandsplatten (19) wählbarer
Stärke zwischen den reflektierenden Oberflächen und den
Flächen des Messkopfes (1) vorgesehen sind.

8. Vorrichtung zur berührungslosen ein- bis dreidimensionalen Vermessung von Kiefer- bzw. Kiefergelenkbewegungen mit Hilfe von Messeinrichtungen, die optoelektronische Signalgeber und Signalempfänger aufweisen, zwischen
denen Lichtsignale direkt oder über zur Messeinrichtung
gehörende Reflektoren übertragen werden, bei der am Unterkiefer des Patienten ein Teil der Messeinrichtung angebracht ist,
d a d u r c h   g e k e n n z e i c h n e t,
dass vom Signalgeber (25) ein Lichtstrahl (26) abgegeben
wird, der auf einen lichtempfindlichen Signalempfänger
(23) mit ein- oder zweiachsiger Positionsempfindlichkeit
quer zur Auftreffrichtung des Lichtstrahls auftrifft, so
dass das Ausgangssignal des Signalempfängers von der
Relativbewegung zwischen Signalgeber und Signalempfänger
quer zur Auftreffrichtung des Lichtstrahls abhängig ist.

9. Vorrichtung nach Anspruch 8,
d a d u r c h   g e k e n n z e i c h n e t ,
dass das Ausgangssignal des Signalempfängers (23) von der
Relativverschiebung des Signalempfängers quer zur Auftreffrichtung des Lichtstrahls linear abhängig ist.

10. Vorrichtung nach Anspruch 8 oder 9,
d a d u r c h   g e k e n n z e i c h n e t ,
dass der Signalempfänger (23) ein positionsempfindlicher
Fotodetektor ist.

11. Vorrichtung nach Anspruch 10,
d a d u r c h   g e k e n n z e i c h n e t ,
dass der positionsempfindliche Fotodetektor ein Quadrantenfotodetektor mit ein- oder zweiachsiger Anordnung ist.

12. Vorrichtung nach Anspruch 10,
d a d u r c h   g e k e n n z e i c h n e t ,
dass der positionsempfindliche Fotodetektor eine kontinuierlich messende ein- oder zweiachsige lineare positionsempfindliche Diode ist.

13. Vorrichtung nach Anspruch 8 oder 9,
d a d u r c h   g e k e n n z e i c h n e t ,
dass der Signalempfänger (23) eine Ladungsverschiebungsvorrichtung enthält.

14. Vorrichtung nach Anspruch 13,
d a d u r c h   g e k e n n z e i c h n e t ,
dass die Ladungsverschiebungsvorrichtung eine ladungsgekoppelte Vorrichtung (CCD) ist.

15. Vorrichtung zur berührungslosen ein- bis dreidimensionalen Vermessung von Kiefer- bzw. Kiefergelenkbewegungen mit Hilfe von Messeinrichtungen, die optoelektronische Signalgeber und Signalempfänger aufweisen, zwischen denen Lichtsignale direkt oder über zur Messeinrichtung gehörende Reflektoren übertragen werden, bei der am Unterkiefer des Patienten ein Teil der Messeinrichtung angebracht ist,
d a d u r c h   g e k e n n z e i c h n e t,
dass von jedem Signalgeber (40) ein Lichtstrahl abgegeben wird, der auf ein reflektierendes oder durchlässiges Gitter (39) auftrifft, das sich relativ zum Signalgeber bewegt, wobei Signalgeber (40) oder Gitter (39) mit dem Unterkiefer verbunden sind und wobei durch die Messeinrichtung keine direkte oder indirekte Kraft zwischen den Signalgebern (40) und den Gittern (39) übertragen wird, so dass die Zahl der vom Gitter reflektierten oder durchgelassenen Lichtimpulse von der Relativbewegung des jeweiligen Signalgebers zu dem zugehörigen Gitter quer zur Auftreffrichtung des Lichtstrahls abhängig ist (inkrementale Wegmessung).

16. Vorrichtung nach Anspruch 14,
d a d u r c h   g e k e n n z e i c h n e t,
dass das reflektierende oder lichtdurchlässige Gitter (39) ein kodiertes Gitter ist.

17. Vorrichtung nach einem der vorhergehenden Ansprüche,
d a d u r c h   g e k e n n z e i c h n e t,
dass der den Lichtstrahl abgebende Sender (8) bzw. der den Lichtstrahl abgebende Signalgeber (25 bzw. 40) eine Leuchtdiode ist und dass der Empfänger (9 bzw. 40) ein Fototransistor ist.

18. Vorrichtung nach einem der vorhergehenden Ansprüche,
d a d u r c h   g e k e n n z e i c h n e t,
dass der einen Lichstrahl abgebende Sender (8) bzw. der
einen Lichtstrahl abgebende Signalgeber (25) ein Laser
ist.

19. Vorrichtung nach einem der vorhergehenden Ansprüche,
d a d u r c h   g e k e n n z e i c h n e t,
dass der Messkopf (1) bzw. die Messplatten bzw. der
Signalgeber (25, 30) mit Hilfe von Drehgelenken (15, 16)
justierbar und arretierbar ist.

Fig. 1

Fig. 2

Fig. 3

Fig. 4

0064687

Fig. 5

Fig. 6

Fig. 7

Fig. 8

µA Differenzstrom

Position
mm

0064687

Fig. 9

y-Position

x-Position

Fig. 10